# EUROPÄISCHE PATENTSCHRIFT

(11) **EP 1 891 918 B1**
(45) Veröffentlichungstag und Bekanntmachung des Hinweises auf die Patenterteilung: **21.07.2010**
(21) Anmeldenummer: 07015662.5
(22) Anmeldetag: 09.08.2007
(51) Int. Cl.: A61F 13/02

(54) **Vorrichtung zur Herstellung von Gipsbinden sowie Verfahren hierzu**
Device for manufacturing plaster bandages and method therefor
Dispositif destiné à la fabrication de bandes de plâtre et procédé correspondant

(30) Priorität: 24.08.2006 DE 102006041656
(43) Veröffentlichungstag der Anmeldung: 27.02.2008
(73) Patentinhaber: Paul Hartmann Aktiengesellschaft, 89522 Heidenheim (DE)
(72) Erfinder: Zoch, Matthias, 89555 Steinheim (DE); Lohrengel, Armin, Dr., 89522 Heidenheim (DE); Ostertag, Wolfgang, 89541 Gerstetten (DE)
(74) Vertreter: Langöhrig, Angelika Beate

(56) Entgegenhaltungen:
- US-A- 2 914 421
- US-A- 3 630 194

## Beschreibung

Die Erfindung betrifft eine Vorrichtung zur Herstellung von Gipsbinden umfassend Mittel zum Transportieren eines bindenartigen Flächengebildes durch die Vorrichtung sowie eine Auftragseinrichtung zum Aufbringen einer Schlämme eines Gipsmaterials auf das Flächengebilde und einen ersten der Auftragseinrichtung nachgeschalteten Ofen zum Trocknen der Schlämme und ein zweiter dem ersten nachgeordneten Ofen zum Calzinieren des Gipsmaterials.

Verfahren und Vorrichtungen zum Herstellen von Gipsbinden sind seit langem bekannt.

Im Wesentlichen wurden dabei in der Vergangenheit zwei Verfahren zur Gipsbindenherstellung verwendet. So wird nach einem ersten Verfahren bereits das in einem vorhergehenden Schritt calzinierte Semihydrat des Gipses (CaSO₄ * ½ H₂O) eingesetzt. Als Ausgangsprodukt kommen dabei sowohl synthetische als auch natürliche Gipsgesteine zum Einsatz. Da das Semihydrat vor Anwendung in dieser Form nicht mehr mit Wasser in Berührung kommen darf, muss das Semihydrat in eine Suspension mit organischem Lösemittel überführt und auf ein Flächengebilde aufgetragen werden. Bei den Lösemitteln handelt es sich in der Regel um explosive und/oder gesundheitsschädliche organische Lösemittel. Daher müssen aufwändige Vorsorgen getroffen werden, von denen insbesondere die Rückgewinnung der Lösemittel mit nicht unerheblichen Kosten verbunden ist. Typischerweise aber nicht ausschließlich kommen für Gipsbinden Methanol und Dichlormethan als Mischungen oder nur Dichlormethan zur Anwendung.

Die Trocknung erfolgt in Gastrocknern bei typischerweise 105°C bis 120°C. Der Trockner und auch das Auftragswerk müssen gasdicht ausgeführt sein, wobei hier abhängig von der Anlage als Trockenmedium Luft oder Stickstoff zum Einsatz kommen. Im Falle des Stickstoff handelt es sich in der Regel um keine explosions-geschützte Anlage, dennoch muss der Trockner und das Auftragswerk gasdicht ausgeführt sein und mit einem Kreislaufsystem zur Aufbereitung des Stickstoffs ausgestattet werden. Das Flächengebilde wird dabei in einem Arbeitsgang beschichtet, getrocknet und aufgewickelt. Die Trocknung verläuft relativ schnell, so dass Verweilzeiten von circa zwei Minuten im Trockner ausreichen. Auch bei diesen Temperaturen ist eine weitere Dehydrierung des Gipswerkstoffs prinzipiell möglich, das heißt eine Reaktion vom Semihydrat zum Anhydrit III. Es werden dann sogenannte Mutterrollen hergestellt, die dann weiter konfektioniert und in wasserdampfundurchlässige Hüllen verpackt werden.

Darüber hinaus existiert ein Verfahren zur Gipsbindenherstellung, in dem das Dihydrat des Calziumsulfats (CaSO₄ * 2 H₂O) in wässriger Suspension eingesetzt wird. Das Verfahren ist vergleichsweise einfach, da keine explosionsgeschützten Anlagen notwendig sind. Hierbei wird im ersten Schritt ein Durchlauftrockner ohne Umlaufsystem mit Luft als Trocknungsmedium eingesetzt. Als Ausgangsstoff kommen hier Calziumsulfat-Dihydrate aus natürlichen und synthetischen Ursprüngen in Frage. Dabei wird beispielsweise das Dihydrat in einer wässrigen Suspension auf ein Flächengebilde aufgetragen und anschließend getrocknet. Die Trocknungszeit beträgt circa zwei Minuten und die trockene Gipsbinde erreicht beim Verlassen des Trockners Temperaturen von 120°C bis 135°C. Der Gips ist gegenüber dem Ausgangsprodukt praktisch unverändert und liegt noch vollständig in Dihydratform vor. Die Gipsbinde wird nach der Trocknung zum Beispiel auf Haspeln in eine Spiralform aufgewickelt, wobei die einzelnen Lagen nicht zu dicht liegen dürfen, um das beim Calzinieren frei werdende Kristallwasser bei der Diffusion nicht zu behindern oder gar wieder als Kristallwasser einzubauen.

In einem zweiten Schritt wird dann die aufgerollte Binde in einen kammerartigen Calzinierungsofen eingebracht und bei circa 160°C bis 170°C calziniert. Hierbei wird das Kristallwasser entfernt. Die Addition der Verweilzeiten beider Prozesse beträgt circa eine Stunde. Allerdings wird neben dem gewünschten Semihydrat auch die nächste Dehydrierstufe, nämlich das unerwünschte Anhydrit gebildet. Anhydrit ist dabei frei von Kristallwasser. Die Reaktionsgeschwindigkeit dieser Folgereaktion nimmt mit steigender Temperatur zu. Im technisch relevanten Temperaturband wird also kein reines Semihydrat erzielt, sondern eine Mischphase. Problematisch ist auch, dass durch das Heizmedium Luft die Temperatur in der Gipsbinde nicht gleichmäßig erhöht, sondern zunächst nur die oberste Schicht erwärmt wird. Das heißt in der Außenschicht laufen bereits die Dehydrierung und damit auch die Anhydritbildung, noch bevor der Gips in der Mitte der Gipsbinde, die spiralig aufgewickelt ist, die notwendige Temperatur für die Calzinierung erreicht. Bei einem derartigen Prozess handelt es sich um einen Batchprozess, der insbesondere hinsichtlich der Anpassung der Parameter bei der Herstellung und Calzinierung problematisch sein kann.

Hierdurch können sich am Ende Schwankungen in der Abbindezeit und damit Probleme in der Anwendung ergeben. Darüber hinaus besitzen Binden nach dem zweiten Verfahren gegenüber dem Lösemittelverfahren den Nachteil, dass ein höherer Trockenausfall gegeben ist, das heißt es fällt eine größere Menge Gips bereits vor Anwendung von der Binde.

Des Weiteren ist beispielsweise aus der DE 198 45 621 A1 eine Vorrichtung und ein Verfahren zur Herstellung von hydraulische Bindemittel enthaltenen Versteifungsmaterialien beschrieben, wobei hier eine angerührte S'chlämme aus Zement und Wasser auf ein Bahnmaterial aufgebracht und mittels Heißluft getrocknet wird. Dabei ist als insbesondere vorteilhaft eine Mikrowellentrocknungseinrichtung beschrieben.

Schließlich offenbart die US-PS 2,914,421 eine Herstellungsmethode für Gipsbinden, bei der der Gips mittels IR-Strahlung getrocknet und calziniert wird. Nachteilig bei dem genannten Verfahren ist, dass die eingestellte Temperatur mit 160°C bis 190°C verhältnismäßig hoch liegt und in diesem Bereich ein erheblicher Anteil Anhydrit III hergestellt wird, wobei diese Gipsmodifikation nur ein langsames Abbinden einer Gipsbinde ermöglicht, die nicht den Wünschen der Verbraucher entspricht.

Es ist daher vorteilhaft, wenn die Calzinierungstemperatur möglichst in dem Bereich liegt, in dem Calziumsemihydrat gebildet wird.

Es ist daher Aufgabe der Erfindung, eine Vorrichtung und ein Verfahren zur Herstellung von Gipsbinden bereitzustellen, das ohne organische Lösemittel arbeitet und eine sichere Einhaltung der Calzinierungstemperatur gewährleistet.

Die Erfindung löst diese Aufgabe durch eine Vorrichtung gemäß Anspruch 1 sowie ein Verfahren gemäß Anspruch 13.

Dabei wird die gattungsgemäße Vorrichtung dahingehend weitergebildet, dass dem ersten Ofen ein zweiter Ofen nachgeschaltet ist zum Calzinieren des Gipsmaterials, wobei mindestens der zweite Ofen mehrere in Transportrichtung nacheinander angeordnete Heizelemente in Form von IR-Strahlerfeldern umfasst, wobei jedes IR-Strahlerfeld ein oder mehrere IR-Strahler aufweist sowie einen Temperatursensor und einen zugeordneten Regler, mittels dem die IR-Strahler regelbar sind. Durch die Regelung der einzelnen IR-Strahlerfelder kann dabei das Temperaturprofil besonders vorteilhaft eingestellt werden.

Insbesondere kann vorgesehen sein, dass der Temperatursensor ein Pyrometer ist, wobei dieser vorzugsweise unter einem Winkel von < 90° zur Fläche des Flächengebildes angeordnet sein kann. Dabei besteht der Winkel < 90° insbesondere in einer Ebene quer zur Transportrichtung.

Dabei ist die Anordnung in einem Winkel von < 90° zur Fläche des Flächengebildes vorteilhaft, da es sich bei entsprechenden Flächengebilden zur Herstellung von Gipsbinden um Mullmaterialien oder ähnliche Gewebe handelt, die eine Porosität aufweisen, beziehungsweise keine glatte einheitliche Oberfläche bilden. Bei einem Pyrometer handelt es sich um ein Instrument zur berührungslosen Temperaturmessung, wobei hier die emittierte Infrarotstrahlung eines Gegenstandes gemessen wird. Das heißt, der Pyrometer bestimmt die Temperatur des Flächengebildes mit dem aufgebrachten Gipsmaterial, so dass eine Regelung der Heizelemente dergestalt erfolgen kann, dass die besonders vorteilhaft erwünschte Temperatur zum Calzinieren auf dem Flächengebilde selbst erreicht wird. Es ist somit keine Übertemperierung notwendig, um zu verhindern, dass womöglich keine ausreichende Calzinierung auf der Gipsbinde erfolgt. Zu diesem Zweck ist ein Regler vorgesehen, der die Temperatur und Strahlungsleistung der IR-Strahler eines IR-Strahlerfeldes jeweils in Abhängigkeit der gemessenen Temperatur durch das einem IR-Strahlenfeld zugeordnete Pyrometer regelt. Durch die Anordnung des Pyrometers in einem Winkel von < 90°, wobei der tatsächliche Winkel insbesondere von der Porosität der Oberfläche des Flächengebildes abhängt, wird erreicht, dass das Pyrometer die Oberfläche des Flächengebildes als einheitliche Fläche wahrnimmt, um so Fehlmessungen, die durch die Porosität entstehen können, zu verhindern. Jedem Pyrometer ist dabei ein Regler zugeordnet, wobei mehrere Regler in einem vereint sein können, der dann die einzelnen Strahlerfelder zeitlich versetzt regelt.

Der Winkel, den das Pyrometer zur Oberfläche des Flächengebildes besitzt, hängt dabei von der Porosität des Trägermaterials ab. Je unebener und poröser die Fläche des Trägermaterials ist, desto kleiner ist der Winkel, um die Unebenheiten und Öffnungen im Trägermaterial für das Pyrometer auszublenden.

Dabei kann vorgesehen sein, dass das Pyrometer in einem Winkel von 10° bis 70°, insbesondere in einem Winkel von 10° bis 50°, vorzugsweise in einem Winkel von 10° bis 30° und insbesondere in einem Winkel von 15° bis 25° zur Fläche des Flächengebildes angeordnet ist. Insbesondere bei derartig kleinen Winkeln zur Fläche des Flächengebildes spielen die Öffnungen und Porositäten des Flächengebildes keine Rolle bei der Temperaturmessung mehr.

Der Einsatz von IR-Strahlung zum Aufheizen und Calzinieren bietet den Vorteil, dass durch die IR-Strahlung die Gipsbinde gleichmäßiger erwärmt wird, als es beispielsweise bei Heißluft möglich wäre. Darüber hinaus lassen sich derartige Strahler auch mit geringem Aufwand individuell regeln und es kann so ein gewünschtes Temperaturprofil über die verschiedenen IR-Strahlerfelder in Transportrichtung des Flächengebildes eingestellt werden. So ist an jedem Ort des Calzinierungsofen die optimale Temperatur während des gesamten Verfahrens möglich. Insbesondere kann während des gesamten Verfahrens die Temperatur kontinuierlich gemessen werden und so eine Anpassung der abgegebenen Heizleistung ständig erfolgen. Es kann auf diese Weise eine besonders gute Gleichmäßigkeit der Temperaturverteilung und damit auch eine gute Gleichverteilung der Qualitätsmerkmale einer erzielten Gipsbinde erreicht werden. Insbesondere kann über die Verwendung von IR-Strahlern auch eine höhere Gleichmäßigkeit der Temperaturverteilung über die Dicke der Gipsbinde erreicht werden. Die Calzinierung ist so weitestgehend unabhängig vom Auftragsgewicht.

Es kann des Weiteren vorgesehen sein, dass auch der erste Ofen, der der Trocknung der Schlämme dient, mindestens zwei in Transportrichtung nacheinander angeordnete Heizelemente, insbesondere in Form von IR-Strahlerfeldern beinhaltet, wobei jedes IR-Strahlerfeld ein oder mehrere IR-Strahler umfasst. Auf diese Weise können die Vorteile der Dehydrierung durch IR-Strahler, die bereits zuvor beschrieben worden sind, auch für den ersten Ofen eingesetzt werden.

Dabei können die Heizelemente des erste Ofens einen oder mehrere Temperatursensoren umfassen, wobei insbesondere jeweils ein Temparatursensor einem IR-Strahlerfeld zugeordnet ist und einen Regler, der die Heizelemente in Abhängigkeit der durch den Sensor gemessenen Temperatur regelt, und besonders bevorzugt kann vorgesehen sein, dass auch dieser Temperatursensor ein Pyrometer ist. Auf diese Weise können auch im Bereich der Trocknung der Schlämme des Gipsmaterials bereits die Temperaturen im Flächengebilde selbst gemessen werden und es ist so eine besonders vorteilhafte Temperaturregelung über die gesamte Ofenlänge möglich. In diesem Fall kann der Pyrometer unter einem Winkel wie zum zweiten Ofen beschrieben zum Flächengebilde ausgerichtet sein.

Generell kann es sich bei den bindenartigen Flächengebilden um Bahnmaterialien bzw. Flachmaterialbahnen handeln, deren Erstreckung in Transportrichtung sehr viel größer als in der Richtung quer dazu ist. Insbesondere kann es sich um flexible Bahnmaterialien handeln. Insbesondere können solche Bahnmaterialien Gewebe, Gewirke, Nonwoven oder andere textile Materialien sein.

Besonders bevorzugt sind dabei Flächengewichte von 300 bis 500 g/m² Auftragsgewicht der trockenen Gipsmasse nach dem Calzinieren auf der fertigen Binde.

Als IR-Strahlerfelder können dabei ein oder mehrere Paare von IR-Strahlern eingesetzt werden, die beidseits des bewegten also zwischen ihnen durchtransportierte Flächengebilde angeordnet sind oder es kann vorgesehen sein, dass ein oder mehrere IR-Strahler mit auf der anderen Seite des bewegten Flächengebildes angeordneten Reflektoren zusammenwirken. Dabei durchdringt die IR-Strahlung die Flächengebilde und wird durch die Reflektoren wieder zurückgeworfen.

Die IR-Strahler können dabei vorteilhafterweise im Nahinfrarotbereich arbeiten, wobei insbesondere gewünscht ist, dass ein breites Wellenlängenspektrum im Nahinfrarotbereich abgedeckt ist. Es kann jedoch auch vorgesehen sein, dass kleinere Ausschnitte aus dem Nahinfrarotbereich ausgewählt werden. Grundsätzlich können zusätzlich auch Wellenlängen anderer Bereiche mit Nahinfrarotwellen kombiniert werden.

Besonders vorteilhaft ist vorgesehen, dass die Öfen eine vertikale Transportrichtung aufweisen. Diese an sich bekannte Anordnung besitzt den Vorteil, dass der Trockenausfall gering gehalten wird, d. h., dass kein oder nur geringfügige Mengen an Gipsmaterial bereits vor Fertigstellung der Gipsbinden wieder vom Trägermaterial abfällt.

Es kann dabei vorgesehen sein, dass beide Öfen nebeneinander oder übereinander angeordnet sind, wobei die Öfen insbesondere bei einer Anordnung übereinander unmittelbar ineinander übergehen können, so dass keine bauliche Grenze zwischen den beiden Öfen existiert. Es kann somit ein Ofen mit zwei verschiedenen Bereichen ausgebildet sein, wobei der eine Bereich der Trocknung und der andere Bereich der Calzinierung dient. In den Öfen können unterschiedliche Temperaturen oder Temperaturprofile eingestellt werden.

Des Weiteren wird die Erfindung durch ein Verfahren, insbesondere zur Durchführung in einer Vorrichtung der vorstehend beschriebenen Art gelöst, bei der folgende kontinuierlich oder quasi-kontinuierlich ablaufenden Schritte vorgenommen werden, nämlich das Auftragen einer Schlämme aus Gipsmaterial auf ein bindenartiges Flächengebilde, das insbesondere flexibel ausgebildet ist, das anschließende Trocknen der Schlämme in einem ersten Ofen und anschließendes Calzinieren des Gipsmaterials in einem zweiten Ofen durch Bestrahlung mit IR-Strahlung, wobei ein IR-Strahlungsprofil über den zweiten Ofen einstellbar und regelbar ist, in dem die Temperatur des mit Gipsmaterial versehenen Flächengebildes gemessen wird. Es erfolgt hierbei wie bereits vorstehend beschrieben, die Messung der Temperatur unmittelbar auf dem Gipsmaterial. Somit erhält man genaue Werte über die tatsächliche Calzinierungstemperatur und keine Werte über die Umgebungstemperatur.

Die Temperaturmessung und -regelung kann kontinuierlich oder zu diskreten Zeitpunkten stattfinden.

Es kann dabei vorgesehen sein, dass die Calzinierungstemperatur des mit dem Gipsmaterial versehenen Flächengebildes zwischen 135° und 175° und vorzugsweise zwischen 142° und 165° und vorzugsweise zwischen 144° und 160° liegt. Bei derartigen Calzinierungstemperaturen wird ein besonders großer Anteil des gewünschten Semihydrats gebildet, das kurze Abbindezeiten bei einer späteren Verwendung der Gipsbinde kleiner 5 Minuten gewährleistet. Bei Abbindezeiten der später verwendeten Gipsbinden unter fünf Minuten, wozu diese mit Wasser benetzt werden und dann beispielsweise auf einen Körperteil eines Patienten aufgebracht werden und dort aushärten, lässt sich eine besonders gute Verarbeitbarkeit und Akzeptanz erkennen.

Es kann dabei vorgesehen sein, dass das Temperaturprofil über die Länge des ersten und/oder zweiten Ofens im Wesentlichen plateauförmig ist. Das Plateau kann hierbei nach der Einlaufphase verhältnismäßig schnell erreicht werden. Grundsätzlich sind jedoch auch andere lineare und nichtlineare Temperaturprofile denkbar.

Es kann vorgesehen sein, dass die Verweilzeit beziehungsweise Durchlaufzeit im zweiten Ofen zwischen 1 und 5, vorzugsweise 1 und 3 und insbesondere 1,5 und 2,5 Minuten beträgt. Auf die vorstehende Weise kann daher die Produktionszeit für Gipsbinden deutlich verringert werden.

Dabei erfolgt die Regelung der Temperatur dergestalt, dass der Temperatursensor die Temperatur des mit Gipsmaterial versehenen Flächengebildes nach Durchlaufen eines IR-Strahlerfeldes misst und auf Grund der Regelabweichung zum vorgegebenen Sollwert die Energiezufuhr zum davor liegenden Infrarotstrahlerfeld regelt.

Dabei kann die Energie in Form von elektrischen Infrarotstrahlern oder in Form von katalytischen Infrarotstrahlern, die mittels Brenngas arbeiten, bereitgestellt werden. Auch kann vorgesehen sein, dass die gleiche Anzahl von Infrarotstrahlern und Reglern vorgesehen ist, oder es können auch mehrere Infrarotstrahler in einen Strahlerfeld durch einen einzigen gemeinsamen Regler geregelt werden oder gegebenenfalls im ersten Ofen sogar mehrere Strahlerfelder, wobei dann sämtliche Infrarotstrahler die gleiche Strahlleistung abgeben.

Es kann des Weiteren vorgesehen sein, dass die Flächengebilde nach dem Calzinieren zu Rollen aufgewickelt und gegebenenfalls zu Binden konfektioniert werden. Hierzu können an sich bekannte Vorrichtungen und Verfahren eingesetzt werden.

Zur besseren Verarbeitbarkeit kann dabei vorgesehen sein, dass die Flächengebilde vor dem Auftragen der Schlämme angefeuchtet werden. Auf diese Weise wird eine Anhaftung der Schlämme auf den Flächengebilden verbessert. Die überschüssige Schlämme kann dann vor Eintritt in den ersten Ofen entfernt werden.

Ein entsprechendes Verfahren dient dabei besonders bevorzugt zur Herstellung medizinischer Gipsbinden, die zur Herstellung von Gipsverbänden an Patienten dienen.

Die Erfindung soll im Folgenden anhand eines Ausführungsbeispiels näher erläutert werden. Dabei zeigen die Figuren der Zeichnung in schematischer Darstellung:
- Figur 1: eine Vorrichtung gemäß der Erfindung,
- Figur 2: einen Ausschnitt aus dem zweiten Ofen in verschiedenen Ansichten.

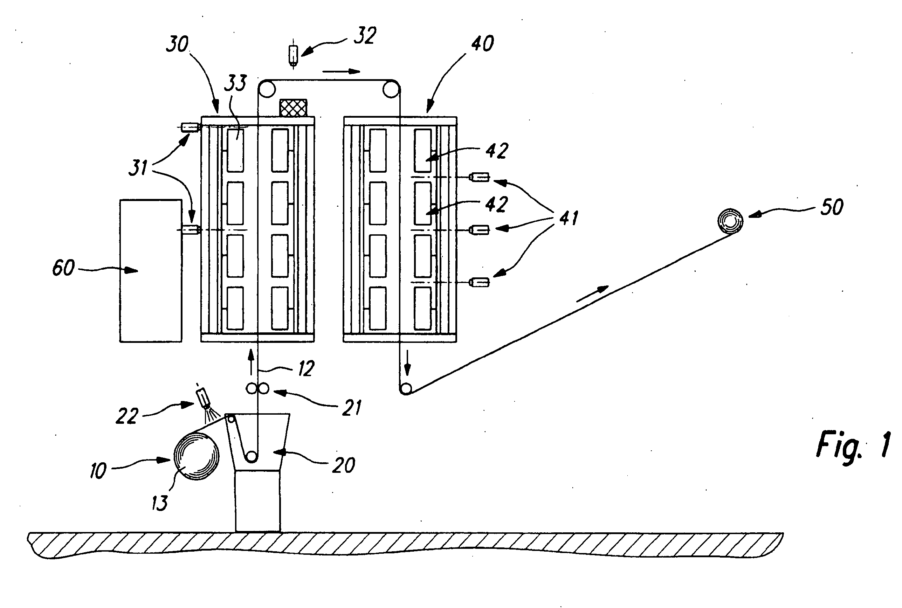

Figur 1 zeigt eine Gesamtanlage, umfassend eine Abwickelvorrichtung, die in der Gesamtheit mit dem Bezugszeichen 10 versehen ist. Hierbei wird ein bindenartiges Flächengebilde 12, das auf einer Rolle 13 aufgewickelt ist, von dieser Rolle abgewickelt und über Führungseinrichtungen durch die Anlage transportiert. Das Flächengebilde 12 wird dabei kontinuierlich abgewickelt und transportiert. Sofern eine Rolle vollständig abgewickelt ist, wird diese ersetzt.

Dabei erfolgt nach dem Abwickeln zunächst ein Befeuchten über eine Befeuchtungsvorrichtung 22 und es wird dann in einem Auftragswerk 20 eine Gipsmaterialschlämme auf das Flächengebilde 12 aufgebracht. Nach dem Auftragswerk 20 durchläuft das Flächengebilde, das von der Rolle 13 abgewickelt worden ist, ein Abstreifwerk 21, das überschüssige Gipsmaterialschlämme abstreift. Hierdurch wird die Auftragsmenge an Gipsmaterial auf dem Flächengebilde eingestellt. Das mit der Gipsmaterialschlämme beschichtete Flächengebilde 12 wird danach in und durch einen ersten Ofen 30 transportiert. Der erste Ofen 30 dient dabei dem Trocknen der Gipsmaterialschlämme, das heißt der Entfernung von Wasser aus der Gipsmaterialschlämme. Die ausreichende Dehydrierung wird über einen Feuchtigkeitsmesser 32, der dem ersten Ofen 30 nachgeschaltet ist, bestimmt.

Der erste Ofen 30 ist hierbei vertikal angeordnet, das heißt das Flächengebilde 12 wird in vertikaler Richtung durch den Ofen 30 transportiert. Beidseits des Flächengebildes, auf die Flächen zugerichtet, sind im ersten Ofen Infrarotstrahler 33 angeordnet, wobei jeweils vier Infrarotstrahler, die zwei Infrarotstrahlerpaare bilden, zu einem Infrarotstrahlerfeld zusammengefasst sind. Je Infrarotstrahlerfeld ist ein Temperatursensor 31 vorgesehen, der als Pyrometer zur berührungslosen Messung der Temperatur in der Gipsmaterialbinde ausgebildet ist. Durch die Temperatursensoren 31 wird nach jedem IR-Strahlerfeld die Temperatur im Gipsmaterial auf dem Flächengebilde 12 ermittelt und es kann so mittels einer Regeleinrichtung, die mehrere Regler umfasst, die schematisch dargestellt und mit 60 bezeichnet ist, eine Regelung der Heizleistung der IR-Strahler 33 erfolgen. Dabei können jeweils die einem Temperatursensor 31 zugeordneten Infrarotstrahler, also jedes IR-Strahlerfeld separat geregelt werden. Die Temperatur des Gipsmaterials in dem ersten Ofen beträgt 100 bis 135° C. Das Wasser, das während des Erhitzens als Gas oder Dampf in dem Ofen vorliegt, kann mittels Absaugeinrichtung aus dem Ofen entfernt werden (hier nicht dargestellt).

Das Flächengebilde 12 verlässt danach den ersten Ofen 30, und wird über eine weitere Transportstrecke in einen zweiten Ofen 42 transportiert, der ebenfalls eine vertikale Ausrichtung besitzt, wobei jedoch hier das Flächengebilde von oben nach unten durch den Ofen geführt wird. Im zweiten Ofen 40, der dem Calzinieren des Gipsmaterials dient, sind vier Infrarotstrahlerpaare 42 angeordnet, wobei hier jedoch ein Infrarotstrahlerpaar zugleich ein IR-Strahlerfeld bildet, dem jeweils ein Temperatursensor 41 zugeordnet ist. Die Temperatursensoren 41 sind ebenfalls als Pyrometer zur berührungslosen Messung der emittierten IR-Strahlung des Gipsmaterials vorgesehen. Jedes IR-Strahlerpaar kann daher durch die Regeleinrichtung 60 angesteuert und bezüglich seiner Heizleistung geregelt werden. Die Verwendung von IR-Strahlern ermöglicht dabei ein besonders gleichmäßiges Aufheizen des Gipsmaterials und darüber hinaus eine gute Temperaturregelung, wobei neben einem plateauartigen Temperaturprofil, das heißt nach einem Einlaufbereich ist die Temperatur des Gipsmaterials im gesamten Ofen gleich, auch andere Temperaturprofile verhältnismäßig problemlos eingestellt werden können.

Alle eingesetzten IR-Strahler sind von der Fa. Krelus zu beziehen, Typ G14 25-2.5, mit einer Leistungsdichte von 50 kW/m² und einer emittierten IR-Strahlung im Wellenlängenbereich von 2,6 bis 9,6 µm. Die eingesetzten Temperatursensoren sind von der Fa. Optex Co., Ltd., Typ Thermo Hunter BA-30TA. Das Gerät arbeitet nach dem Prinzip der Strahlungsthermometrie, d. h., die vom Messobjekt emittierte IR-Strahlung wird von einem Sensor erfasst.

Die Calzinierung findet dabei bei einer Temperatur von ca. 145 bis 160° Celsius statt. Nach dem zweiten Ofen ist dann das Dihydrat in ein Semihydrat umgewandelt worden. Das Kristallwasser, das während des Erhitzens als Gas oder Dampf in dem Ofen vorliegt, kann mittels Absaugeinrichtung aus dem Ofen entfernt werden (hier nicht dargestellt). Über eine weitere Umlenkeinrichtung wird dann das bindenartige Flächengebilde 12 auf einem Aufwickelwerk 50 wieder aufgerollt und einer weiteren Bearbeitung und Konfektionierung zu einsatzbereiten Gipsbinden zugeführt.

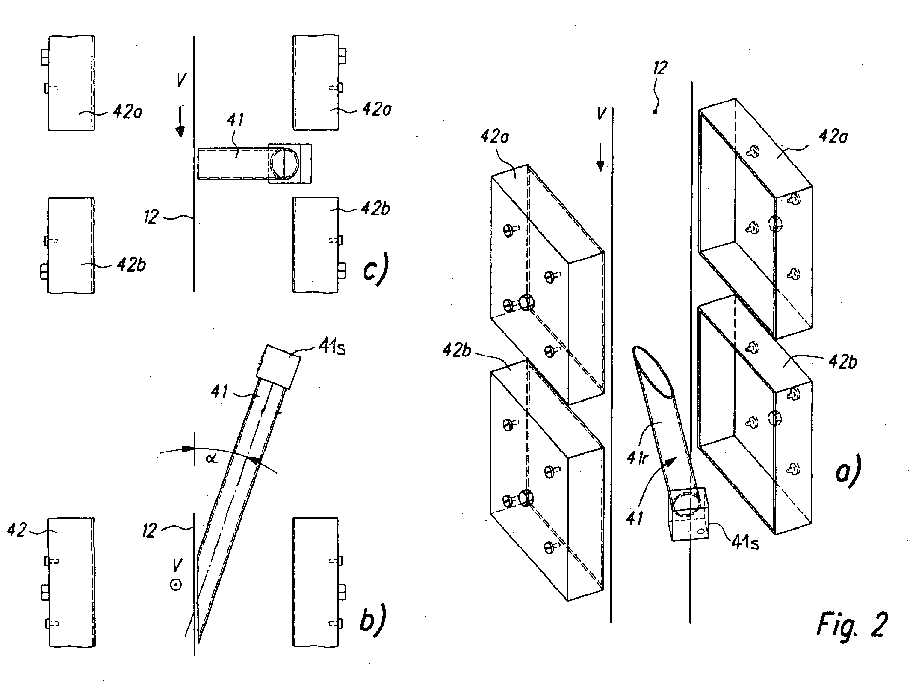

Figur 2 zeigt nun einen Ausschnitt aus dem zweiten Ofen 40 in verschiedenen Ansichten. So ist in Darstellung a) eine perspektivische Darstellung gezeigt. Darstellung b) zeigt eine Darstellung quer zur Transportrichtung und Darstellung c) zeigt eine Ansicht gemäß Figur 1.

In Darstellung a) ist das Flächengebilde 12 perspektivisch von oben nach unten transportierend gezeigt, wobei mit einem Pfeil und dem Bezugszeichen v die Transportrichtung bezeichnet ist. Mit 42 sind hier die IR-Strahlerpaare bezeichnet, wobei mit 42a ein Strahlerpaar und mit 42b ein weiteres Strahlerpaar bezeichnet ist. Jedem der Strahlerpaare ist dem Strahlerpaar nachgeordnet ein Temperatursensor 41 in Form eines Pyrometers zugeordnet, wobei die Darstellung lediglich einen Pyrometer der besseren Anschaulichkeit wegen zeigt.

Der Pyrometer umfasst dabei eine innen und außen schwarz beschichtete Röhre 41r, die unter einem Winkel von ungefähr 20°, wie in der Darstellung b) gezeigt und mit α bezeichnet, zum Flächengebilde 12 ausgerichtet ist. Durch die Röhre 41r wird die vom Flächengebilde 12 emittierte Strahlung aufgenommen und durch den eigentlichen Sensor 41s des Pyrometers, der außerhalb des Ofens 40 angeordnet ist, registriert. Auf diese Weise wird erreicht, dass Porosität und Unebenheiten im Flächengebilde 12 bei der Temperaturmessung keinen Eingang finden. Das Pyrometer 41 ist dabei mit der in Figur 1 mit 60 bezeichneten Regeleinrichtung verbunden, die die Abgabeleistung der Infrarotstrahler 42 regelt und somit für eine gleichmäßige Calzinierungstemperatur beziehungsweise Trocknungstemperatur im zweiten beziehungsweise ersten Ofen sorgt.

Darstellung b) zeigt nun eine Ebene quer zur ersten Darstellung, wobei in Darstellung b) der Winkel zwischen Pyrometer 41 und Materialbahn 12 gesehen werden kann.

Generell ist es wichtig, dass zwischen Materialbahn 12 des Flächengebildes und dem Pyrometer 41 lediglich ein geringer Abstand von 0,2 - 5 cm, vorzugsweise 0,5 - 3 cm, insbesondere 1 bis 3 cm besteht, um eine sichere Temperaturmessung mit wenigen äußeren, das Messergebnis verfälschenden Einflüssen sicherzustellen.

Figur 2c zeigt nun eine Ansicht gemäß Darstellung a), wobei hier keine perspektivische Ansicht gewählt wurde.

In dieser Ansicht weist das Pyrometer einen Winkel von 90° Flächengebilde 12 auf. Durch die entsprechende Anordnung des Pyrometers wird sichergestellt, dass dieses auch durch die Bewegung des Flächengebildes 12 möglichst wenig beeinträchtigt ist.

Auf die vorstehend beschriebene Weise kann erreicht werden, dass besonders vorteilhaft eine gleichmäßige Calzinierungstemperatur bei der Herstellung von Gipsbinden erreicht werden kann, die sicher zu regeln ist. Hieraus resultieren Gipsbinden mit geringen Aushärtezeiten bei einer Verwendung am Patienten sowie geringem Gipsverlust während der Herstellung.

Darüber hinaus kann durch das vorstehende Verfahren und die vorstehend beschriebene Vorrichtung erreicht werden, dass Gipsbinden hergestellt werden können, ohne dass umweltschädliche Lösemittel eingesetzt oder aufwändige, gegebenenfalls sogar explosionsgeschützte Anlagen verwendet werden müssen.

## Patentansprüche

1. Vorrichtung zur Herstellung von Gipsbinden umfassend Mittel zum Transportieren eines bindenartigen Flächegebildes (12) durch die Vorrichtung sowie eine Auftragseinrichtung (10) zum Aufbringen einer Schlämme eines Gipsmaterials auf das Flächengebilde (12) und einen ersten der Auftragseinrichtung (10) nachgeschaltete Ofen (30) zum Trocknen der Schlämme, wobei dem ersten Ofen (30) ein zweiter Ofen (40) nachgeschaltet ist zum Calzinieren des Gipsmaterials, **dadurch gekennzeichnet, dass** mindestens der zweite Ofen mehrere in Transportrichtung nacheinander angeordnete Heizelemente in Form von IR-Strahlerfeldern aufweist, wobei jedes IR-Strahlerfeld einen oder mehrere IR-Strahler (42) umfasst sowie einen Temperatursensor (41), und einen zugeordneten Regler, der die IR-Strahler (42) regelt.

2. Vorrichtung nach Anspruch 1, **dadurch gekennzeichnet, dass** der Temperatursensor ein Pyrometer (41) ist.

3. Vorrichtung nach Anspruch 2, **dadurch gekennzeichnet, dass** der Pyrometer (41) unter einem Winkel von < 90° zur Fläche des Flächengebildes (12) angeordnet ist.

4. Vorrichtung nach Anspruch 3, **dadurch gekennzeichnet, dass** der Pyrometer (41) in einem Winkel von 10-70°, insbesondere in einem Winkel von 10-50°, vorzugsweise in einem Winkel von 10-30° und insbesondere in einem Winkel von 15-25° zur Fläche des Flächengebildes (12) angeordnet ist.

5. Vorrichtung nach einem der vorangehenden Ansprüche, **dadurch gekennzeichnet, dass** der erste Ofen (30) mindestens zwei in Transportrichtung nacheinander angeordnete Heizelemente (33), insbesondere in Form von IR-Strahlerfeldern aufweist, wobei jedes IR-Strahlerfeld einen oder mehrere IR-Strahler umfasst.

6. Vorrichtung nach Anspruch 5, **dadurch gekennzeichnet**, das die Heizelemente (33) des ersten Ofens (30) einen Temperatursensor (31) umfassen, der insbesondere jeweils einem IR-Strahlerfeld zugeordnet ist, und einen Regler, der die Heizelemente (33) regelt.

7. Vorrichtung nach Anspruch 6, **dadurch gekennzeichnet, dass** der Temperatursensor (31) ein Pyrometer ist.

8. Vorrichtung nach einem der vorangehenden Ansprüche, **dadurch gekennzeichnet, dass** die IR-Strahlerfelder ein oder mehrere Paare von IR-Strahlern umfassen, die beidseits eines bewegten Flächengebildes (12) angeordnet sind oder einen oder mehrere IR-Strahler, die mit auf der anderen Seite eines bewegten Flächengebildes (12) angeordneten Reflektoren zusammenwirken.

9. Vorrichtung nach einem der vorangehenden Ansprüche, **dadurch gekennzeichnet, dass** die IR-Strahler im NIR-Bereich arbeiten und insbesondere ein breites Wellenlängenspektrum in diesem Bereich abdecken.

10. Vorrichtung nach einem der vorangehenden Ansprüche, **dadurch gekennzeichnet, dass** die Öfen (30, 40) eine vertikale Transportrichtung aufweisen.

11. Vorrichtung nach einem der vorangehenden Ansprüche, **dadurch gekennzeichnet, dass** die beiden Öfen (30, 40) unmittelbar ineinander übergehen, und insbesondere übereinander angeordnet sind.

12. Vorrichtung nach einem der vorangehenden Ansprüche, **dadurch gekennzeichnet, dass** das Flächengebilde (12) flexibel und/oder aus einem textilen Material, insbesondere ein Gewirke, Gewebe oder Nonwoven ist.

13. Verfahren mit folgenden kontinuierlich oder quasikontinuierlich ablaufenden Schritten, Auftragen einer Schlämme aus Gipsmaterial auf ein bindenartigen Flächengebildes (12), Trocknen der Schlämme in einem ersten Ofen (30), anschließendes Calzinieren des Gipsmaterials in einem zweiten Ofen (40) durch Bestrahlung mit IR-Strahlung, wobei ein IR-Strahlungsprofil über den zweiten Ofen (40) einstell- und regelbar ist, indem die Temperatur des mit Gipsmaterial versehenen Flächengebildes (12) gemessen wird.

14. Verfahren nach Anspruch 13, **dadurch gekennzeichnet, dass** die Calziniertemperatur des mit dem Gipsmaterial versehenen Flächengebildes (12) zwischen 135° und 175° und vorzugsweise zwischen 142° und 165° und besonders vorzugsweise zwischen 145° und 160° liegt.

15. Verfahren nach Anspruch 13 oder 14, **dadurch gekennzeichnet, dass** das Temperaturprofil über eine Länge des zweiten Ofens (40) im Wesentlichen plateauförmig ist.

16. Verfahren nach einem der vorangehenden Ansprüche, **dadurch gekennzeichnet, dass** die Verweil- beziehungsweise Durchlaufzeit im zweiten Ofen (40) zwischen 1 und 5, vorzugsweise 1 und 3 und insbesondere 1,5-2,5 Minuten beträgt.

17. Verfahren nach einem der vorangehenden Ansprüche, **dadurch gekennzeichnet, dass** die Flächengebilde (12) nach dem Calzinieren zu Rollen aufgewickelt und ggf. zu Binden konfektioniert werden.

18. Verfahren nach einem der vorangehenden Ansprüche, **dadurch gekennzeichnet, dass** das Flächengebilde (12) vor dem Auftragen der Schlämme angefeuchtet wird.

19. Verfahren nach einem der vorangehenden Ansprüche, **dadurch gekennzeichnet, dass** die überschüssige Schlämme vor Eintritt in den ersten Ofen (30) entfernt wird.

20. Verfahren nach einem der vorangehenden Ansprüche, **dadurch gekennzeichnet, dass** es zur Herstellung medizinischer Gipsbinden zur Herstellung von Gipsverbänden dient.

21. Verfahren nach einem der vorangehenden Ansprüche zur Durchführung mit einer Vorrichtung gemäß den Ansprüchen 1 - 12.

## Claims

1. Apparatus for producing plaster bandages, comprising means for transporting a bandage-like flat fabric (12) through the apparatus and an application device (10) for applying a slurry of a plaster material to the flat fabric (12) and a first furnace (30) for drying the slurry, said first furnace being located downstream of the application device (10), wherein a second furnace (40) for calcining the plaster material is located downstream of the first furnace (30), **characterised in that** at least the second furnace comprises a plurality of heating elements in the form of IR emitter arrays which are arranged one after the other in the transport direction, wherein each IR emitter array comprises one or more IR emitters (42) and a temperature sensor (41) and an associated controller which controls the IR emitters (42) .

2. Apparatus according to Claim 1, **characterised in that** the temperature sensor is a pyrometer (41).

3. Apparatus according to Claim 2, **characterised in that** the pyrometer (41) is arranged at an angle of < 90° relative to the surface of the flat fabric (12).

4. Apparatus according to Claim 3, **characterised in that** the pyrometer (41) is arranged at an angle of 10-70°, in particular at an angle of 10-50°, preferably at an angle of 10-30° and in particular at an angle of 15-25° relative to the surface of the flat fabric (12).

5. Apparatus according to one of the preceding claims, **characterised in that** the first furnace (30) comprises at least two heating elements (33), in particular in the form of IR emitter arrays, which are arranged one after the other in the transport direction, wherein each IR emitter array comprises one or more IR emitters.

6. Apparatus according to Claim 5, **characterised in that** the heating elements (33) of the first furnace (30) comprise a temperature sensor (31) which in particular is assigned to a respective IR emitter array, and a controller which controls the heating elements (33).

7. Apparatus according to Claim 6, **characterised in that** the temperature sensor (31) is a pyrometer.

8. Apparatus according to one of the preceding claims, **characterised in that** the IR emitter arrays comprise one or more pairs of IR emitters which are arranged on both sides of a moving flat fabric (12) or one or more IR emitters which cooperate with reflectors arranged on the other side of a moving flat fabric (12).

9. Apparatus according to one of the preceding claims, **characterised in that** the IR emitters operate in the NIR range and in particular cover a broad wavelength spectrum in this range.

10. Apparatus according to one of the preceding claims, **characterised in that** the furnaces (30, 40) have a vertical transport direction.

11. Apparatus according to one of the preceding claims, **characterised in that** the two furnaces (30, 40) merge directly into one another and in particular are arranged one above the other.

12. Apparatus according to one of the preceding claims, **characterised in that** the flat fabric (12) is flexible and/or is made from a textile material, in particular a knit, a woven or a nonwoven.

13. Method comprising the following steps which are carried out continuously or quasi-continuously: applying a slurry of plaster material to a bandage-like flat fabric (12), drying the slurry in a first furnace (30), then calcining the plaster material in a second furnace (40) by irradiation with IR radiation, wherein an IR radiation profile across the second furnace (40) can be adjusted and controlled by measuring the temperature of the flat fabric (12) provided with plaster material.

14. Method according to Claim 13, **characterised in that** the calcination temperature of the flat fabric (12) provided with the plaster material lies between 135° and 175° and preferably between 142° and 165° and preferably between 145° and 160°.

15. Method according to Claim 13 or 14, **characterised in that** the temperature profile is substantially flat over a length of the second furnace (40).

16. Method according to one of the preceding claims, **characterised in that** the residence time or run-through time in the second furnace (40) is between 1 and 5, preferably 1 and 3 and in particular 1.5-2.5 minutes.

17. Method according to one of the preceding claims, **characterised in that** the flat fabrics (12), after calculation, are wound to form rolls and are optionally cut to size to form bandages.

18. Method according to one of the preceding claims, **characterised in that** the flat fabric (12) is moistened before the slurry is applied.

19. Method according to one of the preceding claims, **characterised in that** the excess slurry is removed before entering the first furnace (30).

20. Method according to one of the preceding claims, **characterised in that** it is used to produce medical plaster bandages for producing plaster casts.

21. Method according to one of the preceding claims for carrying out using an apparatus according to Claims 1-12.

## Revendications

1. Dispositif pour fabriquer des bandes de plâtre comprenant des moyens pour transporter un objet plat (12) de type bande à travers le dispositif, un dispositif d'application (10) pour appliquer un enduit de plâtre sur l'objet plat (12) et un premier four (30) situé en aval du dispositif d'application (10) pour sécher l'enduit, un deuxième four (40) étant agencé en aval du premier four (30) pour calciner le plâtre, **caractérisé en ce qu'**au moins le deuxième four présente plusieurs éléments de chauffage agencés les uns derrière les autres dans le sens de transport sous forme de champs de rayonnement infrarouge, chaque champ de rayonnement infrarouge comprenant un ou plusieurs rayonnements infrarouges (42) ainsi qu'un capteur de température (41) et un régulateur qui y est associé et qui règle les rayonnements infrarouges (42).

2. Dispositif selon la revendication 1, **caractérisé en ce que** le capteur de température est un pyromètre (41).

3. Dispositif selon la revendication 2, **caractérisé en ce que** le pyromètre (41) est agencé suivant un angle < 90° par rapport à la surface de l'objet plat (12).

4. Dispositif selon la revendication 3, **caractérisé en ce que** le pyromètre (41) est agencé suivant un angle de 10-70°, en particulier suivant un angle de 10-50°, de préférence suivant un angle de 10-30° et en particulier suivant un angle de 15-25° par rapport à la surface de l'objet plat (12).

5. Dispositif selon l'une des revendications précédentes, **caractérisé en ce que** le premier four (30) présente au moins deux éléments de chauffage (33) agencés l'un derrière l'autre dans le sens de transport, en particulier sous forme de champs de rayonnement infrarouge, chaque champ de rayonnement infrarouge comprenant un ou plusieurs rayonnements infrarouges.

6. Dispositif selon la revendication 5, **caractérisé en ce que** les éléments de chauffage (33) du premier four (30) comprennent un capteur de température (31) qui est associé à un champ de rayonnement infrarouge, et un régulateur qui règle les éléments de chauffage (33).

7. Dispositif selon la revendication 6, **caractérisé en ce que** le capteur de température (31) est un pyromètre.

8. Dispositif selon l'une des revendications précédentes, **caractérisé en ce que** les champs de rayonnement infrarouge comprennent une ou plusieurs paires de rayonnements infrarouges, lesquelles sont agencées de chaque côté d'un objet plat (12) en mouvement ou bien un ou plusieurs rayonnements infrarouge qui coopèrent avec des réflecteurs agencés de l'autre côté d'un objet plat (12) en mouvement.

9. Dispositif selon l'une des revendications précédentes, **caractérisé en ce que** les rayonnements infrarouges opèrent dans la zone proche infrarouge (NIR) et couvrent en particulier un large spectre de longueurs d'ondes dans cette zone.

10. Dispositif selon l'une des revendications précédentes, **caractérisé en ce que** les fours (30, 40) présentent un sens de transport vertical.

11. Dispositif selon l'une des revendications précédentes, **caractérisé en ce que** les deux fours (30, 40) se confondent, et en particulier sont agencés l'un au-dessus de l'autre.

12. Dispositif selon l'une des revendications précédentes, **caractérisé en ce que** l'objet plat (12) est flexible et/ou est fabriqué dans une matière textile, en particulier un maillage, un tissu ou un non-tissé.

13. Procédé comprenant de façon continue ou quasi-continue les étapes consistant à appliquer un enduit de plâtre sur un objet plat (12) de type bande, à sécher l'enduit dans un premier four (30), puis à calciner le plâtre dans un deuxième four (40) par irradiation par rayonnement infrarouge, un profil de rayonnement infrarouge pouvant être réglé par le biais du deuxième four (40), et en ce que la température de l'objet plat (12) enduit de plâtre est mesurée.

14. Procédé selon la revendication 13, **caractérisé en ce que** la température de calcination de l'objet plat (12) enduit de plâtre est comprise entre 135° et 175°, de préférence entre 142° et 165°, de façon davantage préférée entre 145° et 160°.

15. Procédé selon la revendication 13 ou 14, **caractérisé en ce que** le profil de température se présente essentiellement sous forme de plateau sur une longueur du deuxième four (40).

16. Procédé selon l'une des revendications précédentes, **caractérisé en ce que** le temps de séjour ou le temps de passage dans le deuxième four (40) est compris entre 1 et 5, de préférence entre 1 et 3 et en particulier entre 1,5 et 2,5 minutes.

17. Procédé selon l'une des revendications précédentes, **caractérisé en ce que** l'objet plat (12) après la calcination est enroulé et le cas échéant confectionné en bandes.

18. Procédé selon l'une des revendications précédentes, **caractérisé en ce que** l'objet plat (12) est humidifié avant l'application de l'enduit.

19. Procédé selon l'une des revendications précédentes, **caractérisé en ce que** l'excédent d'enduit est retiré avant l'entrée dans le premier four (30).

20. Procédé selon l'une des revendications précédentes, **caractérisé en ce qu'**il sert à fabriquer des bandes de plâtre médicales en vue de fabriquer des bandages de plâtre.

21. Procédé selon l'une des revendications précédentes en vue d'une réalisation avec un dispositif selon les revendications 1 à 12.
